# EUROPEAN PATENT APPLICATION

(11) **EP 3 858 238 A1**
(43) Date of publication of application: **04.08.2021**
(21) Application number: 20154904.5
(22) Date of filing: 31.01.2020
(51) Int. Cl.: A61B 5/145, A61B 5/1455, A61B 5/00

(54) **PORTABLE DEVICE AND METHOD FOR PROVIDING TREATMENT DATA TO A USER**

(71) Applicant: trinamiX GmbH, 67063 Ludwigshafen am Rhein (DE)
(72) Inventor: Brill, Jochen, 67063 Ludwigshafen (DE)
(74) Representative: Altmann Stößel Dick Patentanwälte PartG mbB

(57) **Abstract**

The present invention relates to a portable device (110) and a method (210) for providing treatment data to a user (112), to a computer program, and a computer-readable storage medium (144) for performing the method (210). The device (110) and the method (210) may, particularly, be used in the field of body-worn devices, such as wrist-worn devices.

Accordingly, the portable device (110) for providing treatment data to a user (112) wearing the device (110), wherein the treatment data is related to at least one parameter, comprises:
- an optical spectrometer (120) designated for providing spectral information related to the at least one parameter; and
- an evaluation unit (140) configured to
∘ extract at least one value for the at least one parameter by applying a calibration model to the spectral information;
∘ determine treatment data by applying a treatment model to the at least one value for the at least one parameter;
∘ provide the treatment data to the user (112).

## Description

### Field of the invention

The invention relates to a portable device and a method for providing treatment data to a user. The invention further relates to a computer program and a computer-readable storage medium for performing the method according to the present invention. The device and the method may, in particular, be used in the field of body-worn devices, such as wrist-worn devices. However, further fields of application of the present invention may be feasible.

### Prior art

In the last decade, portable devices have become more common in the consumer field to be used for monitoring medical grade vital signs such as blood oxygenation and pulse rate. As a particular advantage, a user can wear the wearable consumer device in a comfortable fashion during daily life activities. By way of example, a consumer device may, typically, comprise an LED emitting light into the skin of a user and a photodiode for measuring reflected photons from the user, wherein the reflected light may show a pulsatile component caused by blood volume variations in the skin and underlying tissues due to heart beat.

US 2019/0374113 A1 discloses a system and a method for use in monitoring application, in particular in photoplethysmograhy (PPG). Herein, the system comprises a light detection unit and an illumination arrangement. The detection unit is configured for collecting light returning from a selected inspection region for determining variations in collected light intensity. The illumination arrangement comprises one or more light sources configured for providing illumination of a selected wavelength range directed toward said inspection region. Wherein the illumination arrangement is arranged around the detection unit to provide symmetrical illumination conditions with respect to an axis connecting said detection unit and said inspection region.

US 1,0213,113 B2 discloses wearable device including a measurement device adapted to be placed on a wrist or ear having a light source with LEDs to measure physiological parameters. The measurement device generates an optical beam having a near infrared wavelength between 700-2500 nanometers by modulating the LEDs, and lenses to deliver the beam to tissue, which reflects the beam to a receiver having spectral filters in front of spatially separated detectors coupled to analog to digital converters that generate at least two receiver outputs. Signal-to-noise ratio of the beam reflected from the tissue is improved by comparing the receiver outputs, and by increasing light intensity from the LEDs. The receiver is synchronized to the modulation of the LEDs and uses a lock-in technique that detects the modulation frequency. The measurement device generates an output signal representing a non-invasive measurement on blood within the tissue.

EP 3 381 368 A1 discloses a method of extracting a glucose feature, a method for monitoring glucose using near-infrared (NIR) spectroscopy and a glucose monitoring device. The methods comprise removing noise from a near-infrared (NIR) data; extracting a glucose signal from the NIR data; and removing temporal drift components from the extracted glucose signal.

EP 3 458 836 A1 discloses a personal hand-held monitor (PHHM) comprising a signal acquisition device for acquiring signals which can be used to derive a measurement of a parameter related to the health of the user, wherein the signal acquisition device comprises a blood photosensor having one or more photo-emitters for transmitting light to a body part of a user, one or more photo-detectors for detecting light transmitted through or scattered by the body part and two or more optical cells, at least one of which contains an analyte to be detected or which mimics the absorption spectrum of the analyte to be detected, through which the light that has been or will be transmitted through or scattered by the body part passes before it reaches the or each photo-detector, wherein the processor of the PHHM is adapted to process the signals received from the or each photo-detector to calculate the difference in intensity of light which has passed through the or each analyte cell and light which has passed through the or each non-analyte cell and to process signals obtained from the photosensor to provide a measurement of the concentration of the analyte in the user's blood, wherein either:
- the or each photo-emitter is a light-emitting diode (LED);
- an optical cell that mimics the absorption spectrum of the analyte to be detected is used and is a manufactured optical filter;
- the PHHM is adapted to provide optical signals at one or more additional wavelengths for transmission to the body part, and the processor of the PHHM is adapted to process signals at the or each additional wavelength to estimate the volume of blood in the field of view of the blood photosensor; or
- there is a ridge on the surface of the signal acquisition device between the photo-emitter(s) and the photo-detector(s).

CN 108 593 593 A discloses a noninvasive blood sugar measuring device adopting serial dual-infrared spectrum detection and analysis, belongs to the fields of optical measurement and novel medical detection devices, and especially relates to non-invasive human body blood sugar detection. The device is mainly composed of a broadband infrared light source, a double-optical filter switcher, an infrared photoelectric sensor, a signal acquiring and processing circuit and the like. The device adopts a transmission spectrum measurement way; a light beam emitted by the broadband infrared light source is irradiated on relevant parts of a human body to be tested, such as fingertips and palms, penetrates through human body tissues, reacts with blood, and then enters the double-optical filter switcher; and the double-optical filter switcher adopts a serial working mode, and allows light beams with two different wavelengths to go through an optical filter and then received by the unit infrared photoelectric sensor. The signal acquiring and processing circuit converts the electrical signal of the infrared photoelectric sensor into a digital signal, and analyzes and calculates the signals of the two different wavelengths to finally obtain human body blood sugar concentration information.

CN 110 575 181 A discloses a method for training a non-invasive blood glucose detection network model based on near infrared spectroscopy (NIR). For a specified number of individual subjects, each individual subject is tested for NIR absorbance, invasive blood glucose concentration, systolic blood pressure, diastolic blood pressure, and pulse rate of human blood glucose at different detection periods, and the collection of ambient temperature and humidity. The above 7 input parameters and the invasive blood glucose concentration values detected for each individual subject daily are used as the sample data time series of the corresponding individual subject on the day, so as to obtain a sample data set of multiple individual subjects. The data is used as training sample data set, a part of the sample data of the individual objects is used as verification sample data set, and the sample data of remaining individual objects is used as the test sample data set. Further, the 7 input parameters and the invasive blood glucose concentration values are used as input variables and target variables to a backpropag-ation (BP) artificial neural network, and the artificial neural network is trained to obtain a network with determined parameters and structure. Further, sensitivity analysis is performed on the basis of the trained BP network, wherein all samples are used for sensitivity analysis, and unimportant variables are excluded according to the analysis results. Further, a new sample data set is established from the screened variables. Part of the sample data of the individual objects is used as the training sample data set, and the sample data of the remaining individual objects is used as the test sample data set. The screened variables and the invasive blood glucose concentration values are used as input variables to a nonlinear autoregressive exogenous model (NARX) network, and the network is trained to obtain a detection model for NIR non-invasive blood glucose concentration detection.

WO 2015/101992 A2 discloses a hand held spectrometer which is used to illuminate the object and measure the one or more spectra. The spectral data of the object can be used to determine one or more attributes of the object. In many embodiments, the spectrometer is coupled to a database of spectral information that can be used to determine the attributes of the object. The spectrometer system may comprise a hand held communication device coupled to a spectrometer, in which the user can input and receive data related to the measured object with the hand held communication device. The embodiments disclosed herein allow many users to share object data with many people, in order to provide many people with actionable intelligence in response to spectral data.

However, for monitoring at least one parameter, specifically a physiological parameter or a pharmaceutical parameter of the user, a separate tool is, typically, used for each kind of parameter. In addition, for obtaining advice with respect to a potential treatment of the user, a further tool is required. As a result, the use of a portable device, such as a body-worn device, is limited to specific circumstances and may exclude persons, such as children, disabled or elderly people, who may be overburdened with deriving treatment advice from at least one value for the at least one parameter communicated to the user. Furthermore, a required treatment may be delayed in time to a point critical for the user.

### Problem addressed by the invention

Therefore, a problem addressed by the present invention is that of specifying a portable device and a method for providing treatment data to a user as well as a computer program and a computer-readable storage medium for performing the method according to the present invention, which at least substantially avoid the disadvantages of known devices and methods of this type.

In particular, it is desirable that the portable device and the related method would be able to provide an efficient monitoring of one or, preferably, more parameters related to the user, specifically physiological parameters or pharmaceutical parameters of the user, wherein it is desirable to provide treatment data to the user by using the portable device without being required to apply a further device for such a purpose. Specifically, it is desirable that the portable device and the related methods are capable of applying a, preferably fully, automatic procedure, thus, allowing a wide-range use of the portable device also for children, disabled or elderly people. Furthermore, it may be desirable that a required treatment may be reported to the user in due time.

### Summary of the invention

This problem is solved by the invention with the features of the independent patent claims. Advantageous developments of the invention, which can be implemented individually or in combination, are presented in the dependent claims and/or in the following specification and detailed embodiments.

As used herein, the expressions "have", "comprise" and "contain" as well as grammatical variations thereof are used in a non-exclusive way. Thus, the expression "A has B" as well as the expression "A comprises B" or "A contains B" may both refer to the fact that, besides B, A contains one or more further components and/or constituents, and to the case in which, besides B, no other components, constituents or elements are present in A.

In a first aspect of the present invention, a portable device for providing treatment data to a user wearing the device is disclosed, wherein the treatment data is related to at least one parameter. Accordingly, the portable device comprises:
- an optical spectrometer designated for providing spectral information related to the at least one parameter; and
- an evaluation unit configured to
   ∘ extract at least one value for the at least one parameter by applying a calibration model to the spectral information;
   ∘ determine treatment data by applying a treatment model to the at least one value for the at least one parameter;
   ∘ provide the treatment data to the user.

As generally used, the term "portable" refers to a property of a device which allows the user at least one of holding, transporting or, preferably, wearing the device. Herein, the more specific terms "wearable" or "body-worn" relate to a property of the device allowing the user to wear the device by attaching the device in a removable fashion to the skin of the user without implanting it into or under the skin of the user. Specifically, the portable device may be a wrist-worn device, in particular a wristwatch, such as a smartwatch. However, attaching the portable device to a different body portion of the user may also be feasible, especially as an ankle band, a bandeau, an ear clip, or a breast band. By way of example, the portable device may also be comprised by a frame used for a smart glass or a video glass. As generally used, the terms "smart glass" or "video glass" refer to a type of glasses which are used in augmented reality or virtual reality settings for displaying data in a visual fashion recognizable by the user of such a glass. As further used herein, the term "user" refers to a person who is actually wearing the device, which comprises that a particular user may remove the device and hand it over to a further person who, after attaching the device to his or her skin, now assumes the position of the user.

According to the present invention, the portable device comprises an optical spectrometer which is designated for providing spectral information related to the at least one parameter. As generally used, the term "information" refers to any kind of data which comprises a content. Herein, the term "data" relates to piece of information that is provided in a digital or digitized form, such as numerical or alphanumerical code. With respect to the present invention, the information may be or comprise "spectral information" which refers to at least one piece of data related to an electromagnetic spectrum, herein also denoted as "spectrum", such a single intensity at at least one particular wavelength, frequency, or photon energy, or a plurality of intensities distributed over at least one selected range of wavelengths, frequencies, or photon energies. Thus, the term "providing information" relates to a process of transmitting a particular piece of information in form of at least one piece of data. With respect to the present invention, the portable device comprises an optical spectrometer which may be used for generating the desired spectral information which is, subsequently, provided to an evaluation unit as further comprised by the portable device.

As generally used, the term "spectrum" refers to a partition of the optical spectral range, especially of the near-infrared (NIR) spectral range as indicated above. Herein, each part of the spectrum is constituted by an optical signal, which is defined by a signal wavelength and the corresponding signal intensity. As generally used, the term "optical" refers to electromagnetic waves having a wavelength of 380 nm to 780 nm and adjoining wavelength ranges, in particular at least a portion of the near infrared (NIR) spectral range. In general, the NIR spectral range is considered to cover wavelengths of 780 nm to 1400 nm. However, the term "optical" is considered herein to cover further wavelengths outside the NIR spectral range, such as other infrared spectral ranges with wavelengths above 1.4 µm, in particular for wavelengths up to 2.6 µm, up to 3.1 µm, up to 3.5 µm, up to 5 µm, up to 5.5 µm, or up 16 µm. Preferably, the wavelengths of 250 nm to 5 µm, preferably of 400 nm to 3 µm, more preferred of 1250 nm to 2.5 µm, are covered by the term "optical" according to the definition as used herein. Thus, the term "light" as used herein, primarily, relates to radiation having a least one wavelength within the indicated wavelength ranges.

As further generally used, the term "optical spectrometer" relates to an apparatus which is capable of acquiring spectral information, wherein the term "acquiring spectral information" refers to recording the signal intensity with respect to the corresponding wavelength of a spectrum or a partition thereof, such as a wavelength interval or at least one individual wavelength, wherein the signal intensity or other kind of spectral information based on the signal intensity can be provided to the evaluation unit for further evaluation. In particular, the spectral information can repeatedly be acquired in-situ.

Preferably, the optical spectrometer may comprise an interferometric filter. As generally used, the "interferometric filter" refers to a device which is designated for separating incident light into a spectrum of constituent wavelength signals whose respective intensities are, subsequently, determined in form of optical signals as generated by a detector array as described below in more detail. Herein, the interferometric filter can, preferably, be selected from at least one Bragg filter; at least one bandpass filter; or a length variable filter, such as a linearly variable filter. As generally used, the term "bandpass filter" refers to an optical element which is designed to transmit a band of wavelengths between two cut-off wavelengths while attenuating outside the band. As an alternative, a so-denoted "band rejection filter" is designed to attenuate in the band while transmitting outside the band. As further generally used, the term "Bragg filter" relates to a particular type of band rejection filter which is comprised by a short segment of a core of an optical waveguide. Herein, a periodic variation has been introduced in the refractive index of the core, whereby a wavelength-specific dielectric mirror has been generated which is designed to attenuate the wavelengths in the band while allowing the wavelengths outside the band to pass undisturbed, thus, acting in a manner similar to a band rejection filter. As further generally used, the term "length variable filter" refers to an optical filter comprising a plurality of interference filters, in particular bandpass filters, which may, particularly, be provided in a continuous arrangement of the filters. Herein, each of the filters may form a bandpass with a variable center wavelength for each spatial position on the filter, preferably continuously, along a single dimension denoted by the term "length" on a receiving surface of the length variable filter. Preferably, the variable center wavelength may be a linear function of the spatial position on the filter, in which case the length variable filter is referred to as a "linearly variable filter". However, other kinds of functions may be applicable to the relationship between the variable center wavelength and the spatial position on the filter. In a particular embodiment, the length variable filter may be a wedge filter, which is designated for carrying at least one response coating on a transparent substrate, wherein the response coating may exhibit a spatially variable property, in particular, a spatially variable thickness. However, other kinds of interferometric filters may also be feasible. In addition, a further optical element which is designed for receiving incident light and transferring it to the interferometric can be used. For further details, reference may be made to WO 2019/115594 A1, WO 2019/115595 A1, or WO 2019/115596 A1.

As an alternative, the optical spectrometer may comprise at least one Fourier-transform infrared spectroscopy (FTIR) spectrophotometer. Herein, the optical spectrometer may comprise at least one broadband light source and at least one interferometric filter, such as a Michelson interferometer. The FTIR spectrophotometer may be configured for providing illumination with at least one light beam having a time-dependent spectrum. For this purpose, the FTIR spectrophotometer may, preferably, comprise at least one moving mirror element, wherein by movement of the mirror element a light beam generated by the broadband light source can alternatingly be blocked and transmitted by the interferometric filter. The optical spectrometer may, furthermore, comprise at least one microelectromechanical system (MEMS) which may be configured for controlling the mirror element. Further, the FTIR spectrophotometer may be configured to modulate the light beam depending on the wavelength such that different wavelengths are modulated at different rates.

Thus, the light may, subsequently, impinge on the detector array. As generally used, the term "detector array" relates to a device comprising a plurality of optical sensors designated for measuring an intensity of the incident light impinging at least one of the optical sensors. Herein, each sensor may, preferably be designated for measuring the intensity of the incident light at a particular wavelength. Therefore, the detector array may, preferably, comprise a sequence of optical sensors that may be located in form of a series of optical sensors one following the other, wherein the sequence of the optical sensors may, preferably, be placed in a parallel manner with respect to the continuous arrangement of the respective optical filters along the length of the length variable filter. Thus, the detector array may, preferably, comprise a series of individual optical sensors which may, in particular, be arranged in a single line as a one-dimensional matrix, preferably along the length of the length variable filter, or in more than one line, especially as two, three, or four lines parallel lines, in form of a two-dimensional matrix, in particular, in order to receive most of the intensity of the incident light as possible. Thus, a number N of pixels in one direction may be higher compared to a number M of pixels in a further direction such that the one-dimensional 1 x N matrix or a rectangular two-dimensional M x N matrix may be obtained, wherein M < 10 and N ≥ 10, preferably N ≥ 20, more preferred N ≥ 50. In addition, the matrixes used herein may also be placed in a staggered arrangement.

Herein, each optical sensor may have the same or, within a tolerance level, a similar optical sensitivity, especially for ease of manufacturing the series of the optical sensors. Alternatively, each of the optical sensors as used in the series of the optical sensors may exhibit a varying optical sensitivity that may vary in accordance with the varying transmittance properties of the length variable filter, such as by providing an increasing variation or a decreasing variation of the optical sensitivity with wavelength along the series of the optical sensors. However, other kinds of arrangements may also be feasible.

In particular, a detector array may be used which may comprise a plurality of pixelated sensors, wherein each of the pixelated sensors is adapted to receive at least a portion of one of the constituent wavelength signals as provided by the length variable filter. As indicated above, each constituent wavelength signal is, hereby, related to an intensity or an amplitude of each constituent wavelength. As generally used, the terms "pixelated optical sensor" or "pixelated sensor" refer to an optical sensor which comprises an array of individual pixel sensors, wherein each of the individual pixel sensors has at least a photosensitive area which is adapted for generating an electrical signal depending on the intensity of the incident light, wherein the electrical signal may, in particular, be provided to the evaluation unit for further evaluation. Herein, the photosensitive area as comprised by each of the individual pixel sensors may, especially, be a single, uniform photosensitive area which is configured for receiving the incident light which impinges on the individual pixel sensor. However, other arrangements of the pixelated sensors may also be conceivable.

The sensor is designed to generate measured optical signals, preferably in form of electronic signals, associated with the intensity of the incident light which impinges on the individual pixelated sensor. The detector signal may be an analogue and/or a digital signal. The electronic signals for adjacent optical sensors can, accordingly, be generated simultaneously, or else in a temporally successive manner. By way of example, during a row scan or line scan, it can be possible to generate a sequence of electronic signals which correspond to the series of the individual optical sensors which are arranged in a line. In addition, the individual sensors may, preferably, be active sensors which may be adapted to amplify the electronic signals prior to providing it to the evaluation unit. For this purpose, the optical sensor may comprise one or more signal processing units, such as one or more filters and/or analogue-digital-converters for processing and/or preprocessing the electronic signals.

The optical sensor may be selected from any known optical sensor, in particular a pixelated sensor, preferably from a pixelated organic camera element, especially a pixelated organic camera chip, or from a pixelated inorganic camera element, especially a pixelated inorganic camera chip, in particular from a CCD chip or a CMOS chip, which are, commonly, used in various cameras nowadays. Herein silicon (Si) can, typically, be used for wavelengths up to 1 µm. As an alternative, especially for wavelengths above 1 µm, the photosensitive area of the optical sensor may comprise a photoconductor, in particular an inorganic photoconductor selected from at least one of indium gallium arsenide (InGaAs), in particular for wavelengths up to 2.6 µm; indium arsenide (InAs), in particular for wavelengths up to 3.1 µm; lead sulfide (PbS), in particular for wavelengths up to 3.5 µm; lead selenide (PbSe), in particular for wavelengths up to 5 µm; indium antimonide (InSb), in particular for wavelengths up to 5.5 µm; and mercury cadmium telluride (MCT, HgCdTe), in particular for wavelengths up 16 µm. However, other photoconductors or further kinds of materials may also be feasible.

Herein, it may particularly be preferred when the spectral sensitivities of the detector array may exhibit a spectral range which may be closely related to an emission spectrum of the light source, particularly to ensure that the detector array may be capable of providing a detector signal having a high intensity, thus, enabling an evaluation of the measured optical signals with sufficient signal-to-noise-ratio and, at the same time, a high-resolution.

Further, the optical spectrometer may comprise further components, such as a light source. Preferably, the light source and the optical spectrometer may be integrated into a single unit, however, they can also be provide as individual units. As used herein, the term "light source" refers to a kind illumination source which is known to provide sufficient emission in the NIR spectral range, preferably within the wavelength ranges as indicated above. As an alternative, a light source may be dispensable in a particular embodiment in which light may be available from surroundings of the portable device sufficient for the optical spectrometer to provide the desired spectral information. For a light source integrated into the portable device, the illumination source may, be selected from at least one of: an incandescent lamp, in particular a light bulb; a laser, in particular a laser diode, although further types of lasers can also be used; a light emitting diode; an organic light source, in particular an organic light emitting diode; a structured light source. However, other kinds of illumination sources can be used, such as a thermal infrared emitter. As used herein, the term "thermal infrared emitter" refers to a micromachined thermally emitting device which comprises a radiation emitting surface that is designated for emitting the desired radiation. By way of example, thermal infrared emitters are available under the name "emirs50" from Axetris AG, Schwarzenbergstrasse 10, CH-6056 Kagiswil, Switzerland, as "thermal infrared emitters" from LASER COMPONENTS GmbH, Werner-von-Siemens-Str. 15 82140 Olching, Germany, or as "infra-red emitters" from Hawkeye Technologies, 181 Research Drive #8, Milford CT 06460, United States. However, further types of thermal infrared emitters may also be feasible.

Herein, the light source may be a continuous light source or, as an alternative, a pulsed light source, wherein the pulsed light source may have a modulation frequency of at least 1 Hz, of at least 5 Hz, of at least 10 Hz, of at least 50 Hz, of at least 100 Hz, of at least 500 Hz, of at least 1 kHz, or more. In a particular embodiment, at least one of the optical spectrometer or the light source can comprise a modulation device designated for modulating the illumination, preferably a periodic modulation. As generally used, a the term "modulation" refers a process in which a total power of the illumination is varied, preferably periodically, in particular with at least one modulation frequency. In particular, a periodic modulation can be effected between a maximum value and a minimum value of the total power of the illumination. The minimum value can be 0, but can also be > 0, such that, by way of example, complete modulation does not have to be effected. Herein, the modulation can, preferably, be effected within the light source designated for generating the desired modulated illumination, preferably, by the light source itself having a modulated intensity and/or total power, for example a periodically modulated total power, and/or by the light source being embodied as a pulsed illumination source, for example as a pulsed laser. As a further example, a device for generating radiation as disclosed in European patent application 19 21 32 77.7, filed December 3, 2019, can also be used for this purpose, wherein the device comprises at least one radiation emitting element, wherein the radiation emitting element is designated for generating radiation upon being heated by an electrical current; a mount, wherein the mount carries the at least one radiation emitting element, and wherein the mount or a portion thereof is movable; and a heat sink, wherein the heat sink is designated for cooling the mount and the at least one radiation emitting element being carried by the mount upon being touched by the mount. Alternatively or additionally, a different type of modulation devices, for example modulation devices based on an electro-optical effect and/or an acousto-optical effect, can also be used. However, a modulation of the light beam at any position within a beam path may also be conceivable, wherein a beam chopper or a different type of periodic beam interrupting device, such as a interrupter blade or interrupter wheel, preferably rotating at constant speed and can, thus, periodically interrupt the illumination, can also be used. Accordingly, the detector array can be designated for detecting at least two measured optical signals in the case of different modulations may have different modulation frequencies. Herein, the evaluation unit as described below in more detail can further be designated for generating the spectral information from the at least two measured optical signals.

Further, the optical spectrometer may comprise at least one optical waveguide which can be designated for guiding at least a portion of the light emitted by the light source towards at least one location at a surface of the skin of the user. Herein, the at least one optical waveguide can be arranged between the light source and the at least one location at the skin of the user. Specifically, light having the same or a different wavelength or wavelength range can be guided to at least one location at the surface of the skin of the user. Herein, a first portion of the light impinging on the surface of the skin of the user may be reflected to return to the device, while a further portion may penetrate a layer of the skin close to the surface of the skin, where it may interact with the skin in a fashion that a share of this portion may be returned to the device. A portion of the light returning to the device can be collected by a transfer element and guided towards the optical spectrometer. Examples of preferred transfer elements can be found in WO 2019/115594 A1, WO 2019/115595 A1, or WO 2019/115596 A1.

Further according to the present invention, the portable device comprises an evaluation unit. As used herein, the term "evaluation unit" refers to an arbitrary device which is designed for further processing of the spectral information as provided by the optical spectrometer in a fashion as described below in more detail. For this purpose, the evaluation unit may be or comprise at least one data processing unit having at least one integrated circuit, such as one or more application-specific integrated circuits (ASICs), and/or one or more digital signal processors (DSPs), and/or one or more field programmable gate arrays (FPGAs), and/or at least one microcomputer and/or microcontroller. Additional components may be comprised, such as one or more preprocessing units, such as one or more devices for receiving and/or preprocessing of the sensor signals, such as one or more AD converters and/or one or more filters. Further, the evaluation unit may, preferably, comprise at least one data storage medium. Further, as outlined above, the evaluation unit may comprise at least one interface, such as a wireless interface and/or a wire-bound interface.

Firstly, the evaluation unit according to the present invention is configured to extract at least one value for the at least one parameter by applying a calibration model to the spectral information. As already indicated above, the term "spectral information" refers to a piece of information that is related to at least one piece of data in relationship to an electromagnetic spectrum. As used herein, the "spectral information" relates to spectral information which is acquired with respect to the least one unknown value for a parameter which is related to the user, whereas the term "reference spectral information" relates to spectral information which refers to a reference value that is known for at least one parameter. As used herein, the term "reference analytical data" refers to at least one piece of data which is related to a known value for the at least one parameter. Accordingly, the reference spectral information and the reference analytical data can, preferably, be used for generating a calibration model. As generally used, the term "calibration model" refers to a model comprising an adjustment of the reference spectral information to the reference analytical data in order to be able to derive analytical data from the spectral information related to a particular parameter by using the model. Herein, a process of adjusting the reference spectral information to the reference analytical data is described by the term "generating the calibration model", while the term "applying the calibration model" denotes a further process of deriving the analytical data from the spectral information related to the at least one parameter.

Herein, the calibration model may, preferably, be implemented by using the at least one parameter, specifically a single parameter or a set of parameters, for a description of the analytical data. Based on the least one parameter, the model is configured to sufficiently represent the adjustment in a reasonable manner, in particular, by resulting in a deviation below a threshold of the adjustment of the reference spectral information to the reference analytical data by solely using the at least one parameter. As generally used, the term "parameter" refers to a logical or a numerical code, depending on a content of the parameter. In particular, the at least one parameter may be a primary parameter, particularly selected from a physical parameter, such as a relative intensity of at least one particular wavelength or wavelength range, also denoted by the term "color", in an optical spectrum, or an indicator whether the intensity at the particular wavelength or wavelength range exceeds an intensity threshold; or a process parameter, such as a time passed since the last acquisition of an optical spectrum, or a particle size distribution as determined from a spectrum. Alternatively or in addition, the at least one parameter may be a secondary parameter, wherein the term "secondary parameter" refers to a type of parameter which is determined from at least one primary parameter. By way of example, the primary parameter may be a relative intensity of at least one particular wavelength or wavelength range in the optical spectrum, whereas the secondary parameter may be a physiological parameter or a pharmaceutical parameter, specifically a content of water, lipid and/or protein in the skin of the user, or a glucose value or a drug concentration in the blood of the user, which may be determined by using a single primary parameter or a combination of at least two different primary parameters. However, further kinds of parameters may also be feasible.

Thus, the term "extracting at least one value for the at least one parameter" as used herein refers to a process of determining at least one value for the at least one parameter by using the calibration model for the adjustment of the spectral information as acquired in an actual measurement. As a result thereof, the corresponding analytical data are sufficiently described by the at least one parameter. Consequently, the at least one parameter can be used as a kind of synopsis for the measured properties. In general, the amount of data used for the at least one parameter constitutes only a small fraction of the amount of data which are required to describe the related spectrum.

In a particularly preferred embodiment, for generating the calibration model at least one learning algorithm which may, preferably, be selected from a machine-learning algorithm or a deep learning algorithm, may be applied. As generally used, the term "learning algorithm" relates to a process of extracting at least one pattern in a plurality of known sets of data, wherein the at least one pattern can, subsequently, be applied for at least one unknown set of data. In addition, by suing further unknown sets of data the at least one pattern can further be refined. In particular, the determining of the treatment data by using the at least one value for the at least one parameter may, preferably, be performed by applying the at least one learning algorithm to a combination of known values for known parameters with known treatment data. Herein, the learning algorithm may involve at least one algorithm selected from a regression algorithm, such as a least square regression; a Bayesian algorithm, a decision tree algorithm, a statistical analysis, a neural network algorithm. However, further kinds of learning algorithms may also be feasible.

Further, the evaluation unit according to the present invention is, additionally, configured to determine treatment data by applying a treatment model to the at least one value for the at least one parameter. As used herein, the term "treatment data" refers to at least one piece of data which is related to a proposed treatment of the user. Thus, the term "determining treatment data" as used herein refers to a process of generating the at least one piece of data related to the proposed treatment of the user from the at least one value for the at least one parameter. As used herein, the term "treatment model" refers to a model comprising a relationship between the treatment data and the at least one value for the at least one parameter in order to derive a particular treatment data from a particular value of the at least one parameter. By way of example, in a case in which a primary parameter selected from a relative intensity of at least one particular wavelength or wavelength range in the optical spectrum may be used, the treatment data can be determined depending on a particular value for the relative intensity. Herein, the relationship between the treatment data and the at least one value for the at least one primary parameter may be comprised by an analytical function, such as a linear, a quadratic or an inverse relation; or by a lookup table indicating the particular treatment data for a particular value or value range of the primary parameter. Similar, in a case in which a secondary parameter may specifically be selected from a content of water, lipid and/or protein in the skin of the user, or a glucose value or a drug concentration in the blood of the user, a glucose value or a drug concentration in the blood of the user may be used, the treatment data can be determined in a similar fashion. However, further kinds of relationships between the treatment data and the at least one value for the at least one parameter may also be feasible. Irrespective whether the parameter may be a primary parameter or a secondary parameter,

As used herein, the term "treatment" refers to a procedure which is recommended for being performed by the user by using the portable device in a fashion that at least one item of information related to the treatment data is provide to the user as described below in more detail. Herein, the treatment may refer to an arbitrary recommended procedure which refers to a state of the body of the user and which can, typically, be performed by the user. In particular, the recommended procedure may be selected from a recommendation regarding nutrition; physical activity, such as movement, exercise, or location; mental activity such as relaxation and/or meditation; sleeping or waking up of the user. By way of example, the treatment may be a recommendation for eating, not eating, drinking, and/or not drinking a particular diet. As a further example, the treatment may be a recommendation for moving the body of the user in general, in a more vehement or slow manner, and/or to or from a particular location, such as out of the sun or towards a medical facility. However, further kinds of treatments may also be feasible.

In a particularly preferred embodiment of the present invention, generating the treatment model may be supported by applying a learning algorithm, preferably selected from a machine-learning algorithm or a deep learning algorithm. Herein, for determining of the treatment data by using the at least one value for the at least one parameter the learning algorithm to a combination of known values for known parameters with known treatment data as known from previous treatments of the user may be applied. For this purpose, known relationships between the treatment data and the at least one value for the at least one parameter may be stored in the storage medium. Alternatively or in addition, the portable device can have a response unit, wherein the term "response unit" refers to an arbitrary means configured for receiving a response by the user, specifically with respect to a particular treatment, as described below in more detail. Herein, the response as provided by the user could be employed when applying the learning algorithm. As a further alternative or in addition, additional information as retrieved via internet or intranet, in particular by employing a data transfer unit as further described below in more detail, could also be used for contributing in generating the treatment model. In particular, the additional information designated for the determining of the treatment data can comprise external data, specifically selected from at least one of: local weather, local weather forecast, GPS data, moon phase, general news. In a particular preferred embodiment, the user's history comprising measured data, external data, and modeled data can be stored as stored data in the database. In a preferred embodiment, the treatment model is or comprises an adaptive model configured to learn to include further parameters. In this preferred, the treatment model can be modified with the at least one item of stored data.

Further, the evaluation unit according to the present invention is, additionally, configured to provide the treatment data to the user. As used herein, the term "providing treatment data" refers to a process of forwarding the at least one piece of data related to the proposed treatment to the user in accordance with the treatment data as indicated below in connection with step d) of the method for providing treatment data to a user. For this purpose, the portable device may, preferably, comprise a user interface which is designated for providing the at least one item of information related to the treatment data to the user. As used herein, the term "user interface" refers to a device which is designated for providing a piece of information, in particular the treatment data, electronically, visually, acoustically or in any arbitrary combination thereof to the user, preferably in a user-receptive manner, most preferred in a user-friendly manner. As generally used, the term "user-receptive manner" relates to a fashion of providing information to a human person such that the human person is capable of comprehending the received piece of information in the desired fashion. For this purpose, the portable device, in particular a wrist-worn device, specifically a wristwatch, such as a smartwatch, may, preferably, comprise at least one display which is designated for providing the at least one item of information related to the treatment data in a visual fashion by displaying it to the user, in particular by at least one of plain text in at least one language or a graphic symbol representing the corresponding piece of information. In addition, the display may comprise a response unit, such as an entry screen, by which a response of the user could be received, specifically with respect to the particular treatment as currently or previously provided to the user. As indicated above, the response provided by the user could be used in further supporting the treatment model, specifically by employing the response in a learning algorithm executed with respect to the treatment model.

Alternatively or in addition, the portable device, in particular a frame being used for a smart glass or a video glass, may be designated for providing the at least one item of information related to the treatment data in a visual fashion by displaying it via the smart glass or the video glass to the user. Alternatively or in addition, the portable device may be designated for providing the at least one item of information related to the treatment data in an acoustic fashion, in particular by comprising at least one acoustic unit, or in a haptic fashion, in particular by comprising at least one haptic unit. In this manner, it can be ensured that the information may reach the user even in an event in which he or she does not observe the display of the portable device. As generally used, the term "acoustic unit" refers to an arbitrary device configured to generate at least one acoustic signal, such as a single tone, a sequence of tones, a melody, or a voice. Similarly, the term "haptic unit" refers to an arbitrary device configured to generate at least one haptic signal, such as at least one impulse or a sequence of impulses designated for impinging the skin of the user.

In a preferred embodiment, the portable device according to the present invention may, further, be configured to keep information in a database stored on a computer-readable storage medium, wherein the storage medium may be comprised by the evaluation unit or may constitute an individual unit separated from the evaluation unit. Herein, the information stored on the database may comprise data related to at least a portion of at least one of: the spectral information, the at least one value for the at least one parameter, the calibration model, the treatment model. However, the database can store further data, such as data related to the portable device and/or the at least one user. By way of example, the database may store data related to the user's history, especially measured data and additional external data, e.g. in a correlated timeline, modeled results and/or a resulting treatment. In general, a long-term monitoring and evaluation of data can be supported by a personal usage of the portable device. As used herein, the term "computer-readable storage medium" may, specifically, refer to a non-transitory data storage means, such as a hardware storage medium having stored there-on data, specifically computer-executable instructions. The computer-readable data carrier or storage medium specifically may be or may comprise a storage medium such as a random-access memory (RAM) and/or a read-only memory (ROM).

In a further preferred embodiment, the portable device according to the present invention may, further, be configured to retrieve additional information available via internet or intranet. For this purpose, the portable device according to the present invention may, further, comprise a data transfer unit, wherein the data transfer unit may be comprised by the evaluation unit or may constitute an individual unit separated from the evaluation unit. As used herein, the term "data transfer unit" refers to an arbitrary device designated for retrieving the additional information the evaluation unit in a wireless fashion. For this purpose, the data transfer unit can, preferably, be selected from at least one of a WLAN receiver and a Bluetooth enabled device. However, a further kind of data transfer unit may also be feasible.

In a further aspect of the present invention, a computer-implemented method for providing treatment data to a user is disclosed. As generally used, the term "computer-implemented method" refers to a method which involves a programmable apparatus, in particular, a computer-readable medium carrying a program, a computer, or a computer network, whereby one or more of the features of the invention are performed by means of at least one program. In accordance with the present invention, the at least one program may be accessible by the computer-readable storage medium comprised by the portable device, or may be provided to the portable device via an in-house network or the internet. With particular regard to the present invention, the present method can, thus, be performed on a programmable apparatus which is configured to this purpose, such as by providing at least one adapted computer program. As a result, the method according to the present invention may, in particular, affect the determining and providing the treatment data to the user, for which purpose the computer-implemented method for providing treatment data to a user as described herein is employed.

The method for providing treatment data to a user as disclosed herein comprises the following steps, which may, preferably, be performed in the given order. Further, additional method steps can be provided which are not listed here. Unless explicitly indicated otherwise, any or all of the method steps, in particular of adjacent method steps, may, at least partially, be performed in a simultaneous manner. Further, any or all of the method steps might be performed at least twice, such as in a repeated fashion, in particular in order to allow repeatedly providing the desired treatment data to the user. Thus, the method for providing treatment data to a user according to the present invention, wherein the user is wearing a portable device, comprises the following steps:
a) providing spectral information related to the at least one parameter;
b) extracting at least one value for the at least one parameter by applying a calibration model to the spectral information;
c) determining treatment data by applying a treatment model to the at least one value for the at least one parameter;
d) providing the treatment data to the user,
wherein steps a) to d) are performed by using the portable device.

According to the present invention, the spectral information can, preferably, be obtained by using measured optical signals related to the at least one parameter. For this purpose, the optical spectrometer as described elsewhere herein, may, preferably, be used. In particular, the method may further comprise evaluating the measured optical signals, wherein the evaluating of the optical signals comprises at least one of: interpolating the optical signals, resampling the optical signals, isolating at least one signal component, analyzing considering non-overlapping windows, normalizing, identifying of peaks. Specifically, the evaluating of the optical signals can be performed by a processing unit which may be comprised by at least one of the optical spectrometer and the evaluation unit.

In a further aspect, the present invention refers to a computer program product. As generally used, the "computer program product" refers to executable instructions for performing the method according to the present invention. For this purpose, a computer program may comprise instructions provided by means of a computer program code which are configured to perform any or all of the steps of the method according to the present invention and, thus, to establish a generation of an image of an object when implemented on a computer or a data processing unit. The computer program code may be provided on a data storage medium or a separate device such as an optical storage medium, e.g. on a compact disc, directly on a computer or data processing unit, or via a network, such as an in-house network or the internet.

Similarly, a computer-readable storage medium is disclosed, comprising instructions which, when executed by the portable device according to the present invention, such as according to any one of the embodiments disclosed above and/or according to any one of the embodiments disclosed in further detail below, cause the portable device to carry out steps a) to d) of the method according to the present invention, such as according to any one of the embodiments disclosed above and/or according to any one of the embodiments disclosed in further detail below.

For further details concerning the computer-implemented method, the computer program product and the computer-readable storage medium, reference may be made to the portable device according to the present invention as disclosed elsewhere herein.

In a further aspect of the present invention, a use of the portable device and the related method according to the present invention is disclosed. Herein, the portable device and the related method is used for a purpose of for providing treatment data to a user, wherein the treatment data may, preferably, be from data related to a recommended procedure selected as described above in more detail.

Summarizing, in the context of the present invention, the following embodiments are regarded as particularly preferred:
Embodiment 1: A portable device for providing treatment data to a user wearing the device, wherein the treatment data is related to at least one parameter; the device comprising:
   - an optical spectrometer designated for providing spectral information related to the at least one parameter; and
   - an evaluation unit configured to
      ∘ extract at least one value for the at least one parameter by applying a calibration model to the spectral information;
      ∘ determine treatment data by applying a treatment model to the at least one value for the at least one parameter;
      ∘ provide the treatment data to the user.
Embodiment 2: The device according to the preceding Embodiment, wherein the optical spectrometer comprises at least one light source designated for illuminating at least one portion of the user.
Embodiment 3: The device according to the preceding Embodiment, wherein the at least one light source is selected from at least one of: an incandescent lamp, in particular a light bulb; a laser, in particular a laser diode; a light emitting diode; an organic light source, in particular an organic light emitting diode; a structured light source.
Embodiment 4: The device according to any one of the two preceding Embodiments, wherein the optical spectrometer further comprises at least one optical waveguide designated for guiding at least a portion of the light emitted by the light source to the user.
Embodiment 5: The device according to any one of the preceding Embodiments, wherein the optical spectrometer further comprises at least one interferometric element designated for receiving the light from the user and separating it into a spectrum of constituent wavelength signals.
Embodiment 6: The device according to the preceding Embodiment, wherein the optical spectrometer further comprises at least one transfer element designated for receiving light from the user and guiding it to the at least one interferometric element.
Embodiment 7: The device according to any one of the preceding Embodiments, wherein the optical spectrometer further comprises at least one detector array designated for generating at least one detector signal.
Embodiment 8: The device according to any one of the preceding Embodiments, wherein the at least one detector array comprises a plurality of pixelated sensors, wherein each pixelated sensor is adapted to receive at least a portion of one of the constituent wavelength signals, wherein each constituent wavelength signal is related to an intensity of each constituent wavelength.
Embodiment 9: The device according to the preceding Embodiment, wherein each pixelated sensor comprises a sensor region, wherein each sensor region comprises a radiation sensitive material.
Embodiment 10: The device according to the preceding Embodiment, wherein the radiation sensitive material is selected from silicon (Si), indium gallium arsenide (InGaAs), indium arsenide (InAs), lead sulfide (PbS), lead selenide (PbSe), indium antimonide (InSb), and mercury cadmium telluride (MCT, HgCdTe).
Embodiment 11: The device according to any one of the two preceding Embodiments, wherein the sensor region is a uniform sensor region.
Embodiment 12: The device according to any one of the three preceding Embodiments, wherein the pixelated sensor is designated for measuring incident light by generating a sensor signal through measuring an electrical resistance or a conductivity of at least a part of the sensor region.
Embodiment 13: The device according to any one of the preceding Embodiments, wherein the evaluation unit comprises a processing device configured to: extract the at least one value for the at least one parameter by applying the calibration model to the spectral information; and to determine the treatment data by applying the treatment model to the at least one value for the at least one parameter.
Embodiment 14: The device according to any one of the preceding Embodiments, wherein the evaluation unit further comprises a user interface designated for providing at least one item of information related to the treatment data to the user.
Embodiment 15: The device according to the preceding Embodiment, wherein the evaluation unit further comprises a driving unit configured to drive the user interface.
Embodiment 16: The device according to the preceding Embodiment, wherein the user interface comprises at least one of: a display designated for displaying at least one item of information related to the treatment data to the user, an acoustic unit designated for providing the at least one item of information to the user in an acoustic fashion, a haptic unit designated for providing the at least one item of information to the user in a haptic fashion.
Embodiment 17: The device according to the preceding Embodiment, wherein the display comprises an entry screen configured for receiving a response by the user, preferably related to the current or previous treatment data.
Embodiment 18: The device according to any one of the preceding Embodiments, wherein the evaluation unit further comprises a computer-readable storage medium configured to store information in a database.
Embodiment 19: The device according to the preceding Embodiment, wherein the computer-readable storage medium is selected from: a random-access memory (RAM), a read-only memory (ROM).
Embodiment 20: The device according to any one of the two preceding Embodiments, wherein the computer-readable storage medium stores data related to at least a portion of at least one of: the spectral information, the at least one value for the at least one parameter, the calibration model, the treatment model.
Embodiment 21: The device according to any one of the preceding Embodiments, wherein the evaluation unit further comprises a data transfer unit configured to retrieve additional information available via internet or intranet in a wireless fashion.
Embodiment 22: The device according to the preceding Embodiment, wherein the data transfer unit is least one of: a WLAN receiver or a Bluetooth enabled device.
Embodiment 23: The device according to any one of the preceding Embodiments, wherein the parameter is selected from at least one primary parameter or at least one secondary parameter.
Embodiment 24: The device according to the preceding Embodiment, wherein the primary parameter is selected from a physical parameter, preferably a relative intensity of at least one particular wavelength or wavelength range, or an indicator whether the intensity at the particular wavelength or wavelength range exceeds an intensity threshold; or a process parameter, preferably a time passed since the last acquisition of an optical spectrum, or a particle size distribution as determined from a spectrum.
Embodiment 25: The device according to any one of the two preceding Embodiments, wherein the secondary parameter is determined from the at least one primary parameter.
Embodiment 26: The device according to any one of the three preceding Embodiments, wherein secondary parameter is selected from a physiological parameter or a pharmaceutical parameter, preferably a content of water, lipid and/or protein in the skin of the user, or a glucose value or a drug concentration in the blood of the user.
Embodiment 27: A computer-implemented method for providing treatment data to a user wearing the device, wherein the treatment data is related to at least one parameter; the method comprising the following steps:
   a) providing spectral information related to the at least one parameter;
   b) extracting at least one value for the at least one parameter by applying a calibration model to the spectral information;
   c) determining treatment data by applying a treatment model to the at least one value for the at least one parameter;
   d) providing the treatment data to the user
   wherein steps a) to d) are performed by using the portable device.
Embodiment 28: The method according to the preceding Embodiment, wherein the portable device is attached to a body part of the user.
Embodiment 29: The method according to the preceding Embodiment, wherein the portable device is attached to the body part of the user by using a wrist band, a frame designed for carrying a smart glass or a video glass, an ankle band, a bandeau, an ear clip, or a breast band.
Embodiment 30: The method according to any one of the preceding Embodiments referring to a method, wherein the spectral information is obtained by using measured optical signals related to the at least one parameter.
Embodiment 31: The method according to the preceding Embodiment, the method further comprising evaluating the measured optical signals, wherein the evaluating of the measured optical signals comprises at least one of: interpolating the optical signals, resampling the optical signals, isolating at least one signal component, analyzing considering non-overlapping windows, normalizing, identifying of peaks.
Embodiment 32: The method according to any one of the preceding Embodiments referring to a method, further comprising generating the calibration model by using reference spectral information and reference analytical data.
Embodiment 33: The method according to the preceding Embodiment, wherein the reference spectral information relates to spectral information which refers to a reference value that is known for at least one parameter, and wherein the reference analytical data refers to at least one piece of data which is related to a known value for the at least one parameter.
Embodiment 34: The method according to any one of the preceding Embodiments referring to a method, further comprising training the treatment model on at least one training dataset, wherein the training dataset comprises a set of treatment data determined by applying the treatment model to the at least one value for the at least one parameter.
Embodiment 35: The method according to any one of the preceding Embodiments referring to a method, wherein at least one of the calibration model and the treatment model is generated by applying a learning algorithm, preferably selected from a machine-learning algorithm or a deep learning algorithm.
Embodiment 36: The method according to any one of the preceding Embodiments referring to a method, wherein the determining of the treatment data by using the at least one value for the at least one parameter is performed by applying the learning algorithm to a combination of known values for known parameters with known treatment data.
Embodiment 37: The method according to any one of the preceding Embodiments referring to a method, wherein the determining of the treatment data comprises using external data, preferably selected from at least one of: local weather, local weather forecast, GPS data, moon phase, general news.
Embodiment 38: The method according to any one of the preceding Embodiments referring to a method, wherein the user's history comprising measured data, external data, and modeled data are stored as at least one item of stored data in a database.
Embodiment 39: The method according to the preceding Embodiment, wherein the treatment model is or comprises an adaptive model configured to learn to include further parameters.
Embodiment 40: The method according to the preceding Embodiment, wherein the treatment model is modified with the at least one item of stored data.
Embodiment 41: The method according to any one of the preceding Embodiments referring to a method, wherein the treatment data comprises at least one item of data related to at least one of: nutrition; physical activity, preferably movement, exercise, or location; mental activity, preferably relaxation and/or meditation; sleeping or waking up of the user.
Embodiment 42: The method according to any one of the preceding Embodiments referring to a method, wherein at least one item of information related to the treatment data is provided to the user via a user interface.
Embodiment 43: The method according to any one of the preceding Embodiments referring to a method, wherein the at least one item of information related to the treatment data is displayed to a user in at least one of: a visual fashion, an acoustic fashion, a haptic fashion.
Embodiment 44: The method according to any one of the preceding Embodiments referring to a method, wherein the spectral information covers a wavelength of 250 nm to 5 µm, preferably of 400 nm to 3 µm, more preferred of 1250 nm to 2.5 µm.
Embodiment 45: A computer program product comprising instructions which, when the program is executed by the device according to any one of the preceding claims referring to a portable device, cause the portable device to carry out steps a) to d) of the method according to any one of the preceding claims referring to a method.
Embodiment 46: A computer-readable storage medium comprising instructions which, when executed by the portable device according to any one of the preceding claims referring to a portable device, cause the portable device to carry out steps a) to d) of the method according to any one of the preceding claims referring to a method.
Embodiment 47: A use of a portable device according to any one of the preceding Embodiments referring to the device portable, for a purpose of providing treatment data to a user wearing the device.

### Brief description of the figures

Further optional details and features of the invention are evident from the description of preferred exemplary embodiments which follows in conjunction with the dependent claims. In this context, the particular features may be implemented alone or with features in combination. The invention is not restricted to the exemplary embodiments. The exemplary embodiments are shown schematically in the figures. Identical reference numerals in the individual figures refer to identical elements or elements with identical function, or elements which correspond to one another with regard to their functions.

### Specifically, in the figures:

- Figure 1: illustrates a preferred exemplary embodiment of a portable device for providing treatment data to a user wearing the device;
- Figure 2: illustrates a diagram indicating a preferred exemplary embodiment of a computer-implemented method for providing treatment data to a user wearing the device.

### Exemplary embodiments

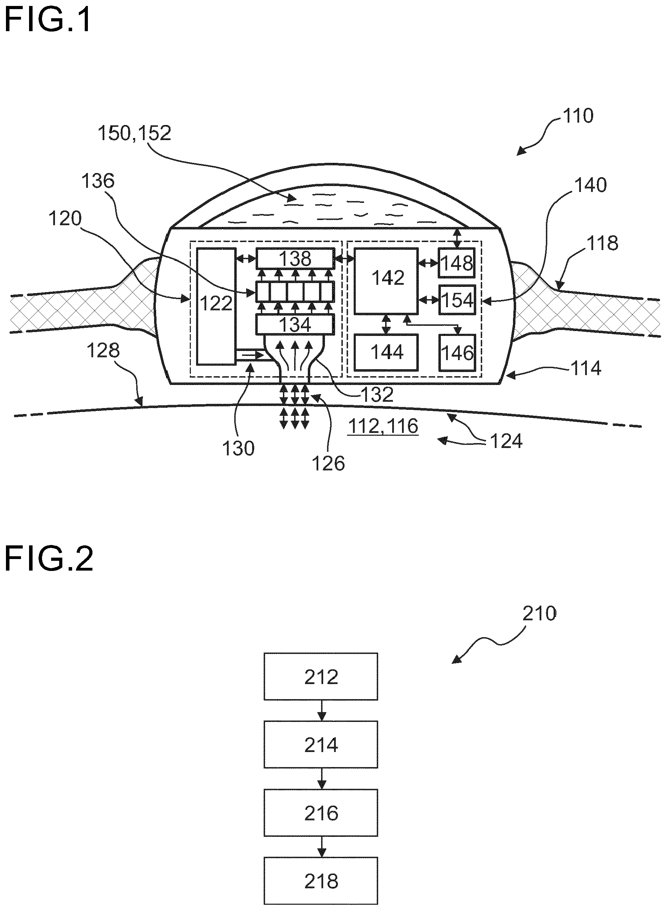

Figures 1 illustrates, in a highly schematic fashion, an exemplary embodiment of a portable device 110 for providing treatment data to a user 112 according to the present invention in a lateral view of a sectional drawing. As schematically depicted there, the device 110 may have a housing 114 which is designated for being worn by the user 112, e.g. at a wrist 116 of the user 112. For this purpose, a wrist band 118 can be employed, wherein the wrist band 116 may, preferably be attached to the housing 114 of the device 110. As a result thereof, the device 110 can be releasably attached to the wrist 116 of the user 112. However, the housing 114 of the portable device 110 may be adapted to be attachable to a further body part of the user 112, in particular by using an ankle band, a bandeau, an ear clip, or a breast band. As an alternative, the portable device 110 may comprise a frame designed for carrying a smart glass or a video glass, wherein the user 112 may wear the frame in a releasably manner similar to eyeglasses.

According to the present invention, the device 110 comprises an optical spectrometer 120 which is designated for providing spectral information related to the at least one parameter. As schematically depicted in Figure 1, the optical spectrometer 120 may, comprise at least one light source 122 which is designated for illuminating at least a portion of a skin 124 of the user 112. In particular, the light source 122 may emit electromagnetic radiation which covers at least a portion of the near infrared (NIR) spectral range. In general, the NIR spectral range is considered to cover wavelengths of 780 nm to 1400 nm. However, the light source 122 may also be capable of emitting further wavelengths outside the NIR spectral range, such as the visible spectral range which covers wavelengths of 380 nm to 780 nm, or in other infrared spectral ranges with wavelengths above 1.4 µm, in particular for wavelengths up to 2.6 µm, up to 3.1 µm, up to 3.5 µm, up to 5 µm, up to 5.5 µm, or up 16 µm. Preferably, the spectral information covers a wavelength of 250 nm to 5 µm, preferably of 400 nm to 3 µm, more preferred of 1250 nm to 2.5 µm.

For a purpose of the generating the desired radiation, the light source 122 may, preferably, comprise a laser diode or an light emitting diode. As an alternative, a thermal radiation source, specifically an incandescent lamp, in particular a light bulb or a thermal infrared emitter as described above in more detail, may also be used. However, a different type of light source 122 may also be feasible. As already indicated above, the light source 122 may be a continuous light source or, as an alternative, a pulsed light source, wherein the pulsed light source may have a modulation frequency of at least 1 Hz, of at least 5 Hz, of at least 10 Hz, of at least 50 Hz, of at least 100 Hz, of at least 500 Hz, of at least 1 kHz, or more. As a result, the modulation frequency, thus, neatly fits with a range of detectivity of infrared sensors which are particularly sensitive at 500 Hz or above, especially due to a strong impact of 1/f noise. For this purpose, a pulsable infrared source comprising a low thermal-mass filament of Tungsten or NiCr can be used. By way of example, such kind of pulsable infrared source is available from Helioworks' EP-Series or EF-Series (refer to www.helioworks.com), or as FLIR from ICx Photonics (refer to www.amstechnologies.com/fileadmin/amsmedia/downloads/2533_IR_Broadband_Sources.pdf). As described above in more detail, a device for generating radiation as disclosed in European patent application 19 21 32 77.7, filed December 3, 2019, can also be used.

As further illustrated in Figure 1, the light emitted by the light source 122 can be guided towards a location 126 at a surface 128 of the skin 124 of the user 112 by an optical waveguide 130, which can be arranged between the light source 122 and the skin 124 of the user 112. In addition, at least one further optical waveguide (not depicted here) can also be used for guiding light having the same or a different wavelength or wavelength range to at least one further location at the surface 128 of the skin 124 of the user 112.

As further depicted in Figure 1, a first portion of the light impinging on the surface 128 of the skin 124 of the user 112 may be reflected to return to the device 110, while a further portion may penetrate a layer of the skin 124 close to the surface 128 of the skin 124, where it may interact with the skin 124 in a manner that a share of this portion may be returned to the device 110. A portion of the light returning to the device 110 can be collected by a transfer element 132 and guided towards the optical spectrometer 120.

For a purpose of generating an optical spectrum from the light received from the skin 124 of the user 112, the optical spectrometer 120 may further comprise an interferometric element 134, which is designated for receiving the light from the skin 124 of the user 112 and separating it into a spectrum of constituent wavelength signals, and a detector array 136 which may comprise a plurality of pixelated sensors, wherein each pixelated sensor is adapted to receive at least a portion of one of the constituent wavelength signals, wherein each constituent wavelength signal is related to an intensity of each constituent wavelength, and to generate at least one detector signal.

Herein, the interferometric element 134 is used in the optical spectrometer 120 for separating the light received from the skin 124 of the user 112 into a spectrum of constituent wavelength signals such that only a single wavelength or a narrow wavelength range may impinge on at least one, preferably exactly one, pixelated sensor as comprised by the detector array 136, wherein respective intensities or amplitudes are determined. Preferably, the interferometric element 134 may be an interferometric filter selected from at least one Bragg filter; at least one bandpass filter; or a length variable filter, such as a linearly variable filter.

As an alternative, the optical spectrometer 120 may comprise at least one Fourier-transform infrared spectroscopy (FTIR) spectrophotometer. Herein, the light source 122 may, preferably, comprise at least one broadband light source and at least one interferometric filter, such as a Michelson interferometer. The FTIR spectrophotometer may be configured for illuminating the skin 124 of the user 112 with at least one light beam having a time-dependent spectrum. Preferably, the FTIR spectrophotometer may comprise at least one moving mirror element, wherein by movement of the mirror element a light beam generated by the broadband light source 122 can alternatingly be blocked and transmitted by the interferometric filter. The optical spectrometer 120 may furthermore comprise at least one microelectromechanical system (MEMS) being configured for controlling the mirror element. Further, the FTIR spectrophotometer may be configured for modulating the light beam depending on the wavelength such that different wavelengths are modulated at different rates.

Further, each pixelated sensor as comprised by the detector array 136 may comprise a uniform sensor region designated for receiving the light from the skin 124 of the user 112 and split into a spectrum of constituent wavelength signals by the interferometric element 134 in a manner that a generation of at least one detector signal may be triggered. Preferentially, the generation of the at least one detector signal may be governed by a defined relationship between the detector signal and the manner of the illumination of the sensor region. Herein, the sensor region may have a size of 2 mm x 2 mm or less, preferred of 1 mm x 1 mm or less. For a purpose of generating the at least one detector signal upon illumination, the sensor region may comprise a radiation sensitive material which can, preferably be selected from silicon (Si), in particular for wavelengths up to 1 µm. For wavelengths above 1 µm, the radiation sensitive material may be selected from at least one of indium gallium arsenide (InGaAs), in particular for wavelengths up to 2.6 µm; indium arsenide (InAs), in particular for wavelengths up to 3.1 µm; lead sulfide (PbS), in particular for wavelengths up to 3.5 µm; lead selenide (PbSe), in particular for wavelengths up to 5 µm; indium antimonide (InSb), in particular for wavelengths up to 5.5 µm; and mercury cadmium telluride (MCT, HgCdTe), in particular for wavelengths up 16 µm. However, other materials may also be feasible for being used in the detector array 136.

As illustrated in Figure 1, the optical spectrometer 120 may, further, comprise an internal processing unit 138, which is designated for determining the desired spectral information by evaluating the measured optical signals as provided by the detector array 136. Herein, the spectral information can, preferably, be obtained by using the measured optical signals related to the at least one parameter. In particular, the evaluating of the measured optical signals which can, preferably, be performed by the processing unit 138 may comprise at least one of: interpolating the optical signals, resampling the optical signals, isolating at least one signal component, analyzing considering non-overlapping windows, normalizing, identifying of peaks. As an alternative, the measured optical signals in the form as provided by the detector array 136 may be used as the desired spectral information, in which event the evaluating of the measured optical signals may be performed later.

According to the present invention, the device 110 comprises an evaluation unit 140 which is configured to
∘ extract at least one value for the at least one parameter by applying a calibration model to the spectral information;
∘ determine treatment data by applying a treatment model to the at least one value for the at least one parameter;
∘ provide the treatment data to the user 112.

For this purpose, the evaluation unit 140 may comprise a processing unit 142, wherein the processing unit 142 may have one or more integrated circuits, such as one or more application-specific integrated circuits (ASICs), and/or one or more digital signal processors (DSPs), and/or one or more field programmable gate arrays (FPGAs), and/or one or more microcomputers and/or microcontrollers. Additional components may be comprised, such as one or more preprocessing units, such as one or more devices for receiving and/or preprocessing of the sensor signals, such as one or more AD converters and/or one or more filters.

As further depicted in Figure 1, the evaluation unit 140 may, further, comprise a computer-readable storage medium 144, wherein the storage medium 144 is configured to store information in a database. Herein, the information stored on the database may comprise data related to the spectral information, reference spectral information, the reference analytical data and/or at least one parameter or at least one value thereof. Furthermore, the database may comprise data related to the user's history, especially measured data and additional external data, e.g. in a correlated timeline, modeled results and/or a resulting treatment. Herein, the user's history comprising measured data, external data, and modeled data can be stored as stored data in a database in the storage medium 144. Preferably, the treatment model may be or comprise an adaptive model configured to learn to include further parameters. Herein, the treatment model can be modified with the at least one item of the stored data. Specifically, the storage medium 144 can comprise a non-transitory data storage means, such as a hardware storage medium having stored there-on computer-executable instructions, specifically selected from a random-access memory (RAM) and/or a read-only memory (ROM). However, a further kind of storage medium 144 may also be feasible.

As further illustrated in Figure 1, the evaluation unit 140 may, further, comprise a data transfer unit 146, which may be configured to retrieve additional information available via internet or intranet in a wireless fashion. For this purpose, the data transfer unit 146 can, preferably, be selected from at least one of a WLAN receiver and a Bluetooth enabled device. However, a further kind of data transfer 146 unit may also be feasible. As described above in more detail, the additional information can, preferably, be used for contributing to the generation of the treatment model. In particular, the additional information designated for the determining of the treatment data can comprise external data, specifically selected from at least one of: local weather, local weather forecast, GPS data, moon phase, general news.

Further according to the present invention, the evaluation unit 140 is, additionally, configured to provide the treatment data to the user. For this purpose, the exemplary evaluation unit 140 as depicted in Figure 1 comprises a driving unit 148 which is configured to drive a user interface 150, wherein the user interface 150 is designated for providing a piece of information, in particular related to the treatment data, electronically, visually, acoustically or in any arbitrary combination thereof to the user 112, preferably in a user-receptive manner, most preferred in a user-friendly manner. For this purpose, the portable device 110 as depicted in Figure 1 comprises a display 152, such as a screen, which is designated for providing the at least one item of information related to the treatment data in a visual fashion by displaying it to the user 112, in particular by at least one of plain text in at least one language or a graphic symbol representing the corresponding piece of information. In a particular embodiment, the display 152 may comprise an entry screen, which can be used as a response unit for receiving a response of the user 112, specifically with respect to a particular treatment currently or previously provided to the user 112. As described above in more detail above, the response as provided by the user 112 could be used for further supporting the treatment model, specifically by employing the response of the user 112 in a learning algorithm as being executed with regard to the treatment model.

In an alternative embodiment (not depicted here), the portable device 110 may comprise a frame which may be used for a smart glass or a video glass. In this particular embodiment, the user interface 150 may be designated for providing the at least one item of information related to the treatment data in a visual fashion by displaying it via the smart glass or the video glass to the user 112.

Alternatively, or in addition, the portable device 110 may, further, comprise at least one acoustic unit 154, wherein the acoustic unit 154 may be designated for providing the at least one item of information related to the treatment data in an acoustic fashion, in particular by emitting acoustic signals and/or at least one voice in one or more languages. In this manner, it can be ensured that the information may reach the user 112 even in an event in which the user 112 may not observe the display 152 of the portable device 110. For a similar purpose, the portable device 110 may, alternatively or in addition, comprise a haptic unit (not depicted here) configured to generate at least one haptic signal, such as at least one impulse or a sequence of impulses designated for impinging the skin of the user 110.

As further depicted in Figure 1 by arrows indicating a single direction or two opposing directions, the evaluation unit 140 may comprise at least one interface, such as a wireless interface and/or a wire-bound interface, which are designated for an exchange of information between the optical spectrometer 120 and the evaluation unit 140 and/or within the optical spectrometer 120 and/or within the evaluation unit 140.

Figure 2 illustrates, in a highly schematic fashion, a computer-implemented method 210 for providing treatment data to a user wearing the device 110. For this purpose, steps a) to d) of the method 210 are performed by using the portable device 110.

In a providing step 212 according to step a), the spectral information related to the at least one parameter is provided by the optical spectrometer 120 to the evaluation unit 140. For spectral information reference can be made to the description above.

In a parametrization step 214 according to step b), the calibration model is applied to the spectral information. In this manner, at least one value for the at least one parameter is extracted from the particular spectral information, preferably by using the processing unit 142, for which purpose the reference spectral information and the reference analytical data, which may, preferably, be stored in the storage medium 144 can be used. Preferably, the at least one parameter as extracted from the particular spectral information, may be stored in the storage medium 144, in particular for immediate use in the following step c).

In a determining step 216 according to step c), treatment data is determined by using the at least one value for the at least one parameter as, preferably, provided by the storage medium 144 to the processing unit 142. As already indicated above, the treatment data may, preferably, comprise at least one item of data related to at least one of: nutrition; physical activity, preferably movement, exercise, or location; mental activity, preferably relaxation and/or meditation; sleeping or waking up of the user.

In an information step 218 according to step d), the treatment data is provided to the user. For this purpose, the evaluation unit 140 may comprise the driving unit 148 which is configured to drive the user interface 150, wherein the user interface 150 is designated for providing a piece of information, in particular related to the treatment data, electronically, visually, acoustically or in any arbitrary combination thereof to the user 112, preferably in a user-receptive manner, most preferred in a user-friendly manner. For this purpose, the portable device 110 may comprise the display 152, such as a screen. Alternatively, or in addition, the acoustic unit 154 may provide the at least one item of information 174 to the user in an acoustic fashion.

### List of reference numbers

- 110: (portable) device
- 112: user
- 114: housing
- 116: wrist
- 118: wrist band
- 120: optical spectrometer
- 122: light source
- 124: skin
- 126: location
- 128: surface
- 130: optical waveguide
- 132: transfer element
- 134: interferometric element
- 136: detector array
- 138: processing unit
- 140: evaluation unit
- 142: processing unit
- 144: storage medium
- 146: data transfer unit
- 148: driving unit
- 150: user interface
- 152: display
- 154: acoustic unit
- 210: computer-implemented method for providing treatment data to a user
- 212: providing step
- 214: parametrization step
- 216: determining step
- 218: information step

## Claims

1. A portable device (110) for providing treatment data to a user (112) wearing the device (110), wherein the treatment data is related to at least one parameter; the device (110) comprising:
- an optical spectrometer (120) designated for providing spectral information related to the at least one parameter;
- an evaluation unit (140) configured to
∘ extract at least one value for the at least one parameter by applying a calibration model to the spectral information;
∘ determine treatment data by applying a treatment model to the at least one value for the at least one parameter;
∘ provide the treatment data to the user (112).

2. The device (110) according to the preceding claim, wherein the optical spectrometer (120) comprises at least one light source (122) designated for illuminating at least one portion of the user (112).

3. The device (110) according to any one of the preceding claims, wherein the optical spectrometer (120) further comprises at least one transfer element (132) designated for receiving light from the user (112).

4. The device (110) according to any one of the preceding claims, wherein the evaluation unit (140) comprises a user interface (150) designated for providing at least one item of information related to the treatment data to the user (112).

5. The device (110) according to the preceding claim, wherein the user interface (150) comprises at least one of: a display (152) designated for displaying at least one item of information related to the treatment data to the user, or an acoustic unit 154 designated for providing the at least one item of information to the user (112) in an acoustic manner.

6. The device (110) according to any one of the preceding claims, wherein the evaluation unit (140) further comprises a computer-readable storage medium (144) configured to store information in a database.

7. The device (110) according to any one of the preceding claims claim, wherein the storage medium (144) stores data related to at least a portion of at least one of: the spectral information, the at least one value for the at least one parameter, the calibration model, the treatment model.

8. The device (110) according to any one of the preceding claims claim, wherein the evaluation unit (140) further comprises a data transfer unit (146) configured to retrieve additional information available via internet or intranet in a wireless fashion.

9. A computer-implemented method (210) for providing treatment data to a user (112) wearing a portable device (110), wherein the treatment data is related to at least one parameter; the method (210) comprising the following steps:
a) providing spectral information related to the at least one parameter;
b) extracting at least one value for the at least one parameter by applying a calibration model to the spectral information;
c) determining treatment data by applying a treatment model to the at least one value for the at least one parameter;
d) providing the treatment data to the user (112),
wherein steps a) to d) are performed by using the portable device (110).

10. The method (210) according to the preceding claim, wherein the spectral information is obtained by using measured optical signals related to the at least one parameter.

11. The method (210) according to the preceding claim, the method further comprising evaluating of the measured optical signals, wherein the evaluating of the measured optical signals comprises at least one of: interpolating the optical signals, resampling the optical signals, isolating at least one signal component, analyzing considering non-overlapping windows, normalizing, identifying of peaks.

12. The method (210) according to any one of the preceding claims referring to the method (210), further comprising generating the calibration model by using reference spectral information and reference analytical data.

13. The method (210) according to any one of the preceding claims referring to the method (210), further comprising training the treatment model on at least one training dataset, wherein the training dataset comprises a set of treatment data determined by applying the treatment model to the at least one value for the at least one parameter.

14. A computer program product comprising instructions which, when the program is executed by the device according to any one of the preceding claims referring to the portable device (110), cause the portable device (110) to carry out steps a) to d) of the method (210) according to any one of the preceding claims referring to a method (210).

15. A computer-readable storage medium (144) comprising instructions which, when executed by the portable device according to any one of the preceding claims referring to the portable device (110), cause the portable device (110) to carry out steps a) to d) of the method (210) according to any one of the preceding claims referring to a method (210).
